# EUROPEAN PATENT SPECIFICATION

(11) **EP 4 052 751 B1**
(45) Date of publication and mention of the grant of the patent: **13.09.2023**
(21) Application number: 22157229.0
(22) Date of filing: 17.02.2022
(51) Int. Cl.: A61M 16/12, A61M 16/20, F01N 1/00, F16K 47/00

(54) **APPARATUS FOR FORCED VENTILATION OF PATIENTS AND RELATED FORCED VENTILATION SYSTEM**
GERÄT ZUR ZWANGSBEATMUNG VON PATIENTEN UND ENTSPRECHENDES ZWANGSBEATMUNGSSYSTEM
APPAREIL POUR LA VENTILATION FORCÉE DES PATIENTS ET SYSTÈME DE VENTILATION FORCÉE CORRESPONDANT

(30) Priority: 01.03.2021 IT 202100004643
(43) Date of publication of application: 07.09.2022
(73) Proprietor: Intersurgical S.P.A., 41037 Mirandola, Modena (IT)
(72) Inventor: PALTRINIERI, Andrea, 41037 Mirandola, Modena (IT)
(74) Representative: Corradini, Corrado

(56) References cited:
- EP-A1- 1 852 137
- EP-A1- 1 985 328
- GB-A- 2 570 511
- US-A1- 2016 184 539
- US-A1- 2016 199 605

## Description

### TECHNICAL FIELD

The present invention relates to an apparatus for the forced ventilation of patients and a system for the forced respiration of patients which incorporates said apparatus.

In particular, the invention relates to an apparatus for continuous positive pressure mechanical ventilation (CPAP) or for non-invasive ventilation (NIV).

### PRIOR ART

As is known, systems for artificial ventilation such as non-invasive ventilation (NIV) or continuous positive airway pressure mechanical ventilation (CPAP), of patients in general, comprising various types of patient interfaces, such as masks or helmets which, in general, are adapted to be coupled sealingly with the face or neck of a patient have been used.

Such systems for artificial ventilation are used in order to allow breathing patients who would not otherwise be able to breathe independently.

For that purpose, through the patient interface, the patients are administered a mixture of breathable gases, e.g. air or oxygen-enriched air or pure oxygen, having a positive pressure that pushes such mixture into the lungs.

During therapy, the patient therefore has his nose and mouth enclosed within the closed volume defined by the patient interface.

Ventilation devices are known which, connected to the patient interface, make respiratory therapy possible. An example of such ventilation devices is shown in the patent application nr. EP 1852137 A1.

For example, for the delivery of the mixture of air and oxygen it is known to use a venturi-effect oxygen-air mixer, also called venturi meter, which uses a flow of oxygen to suck in a flow of air and therefore to mix sucked air with oxygen and deliver the mixture to the patient via the patient interface.

The patient interface, then, at the outflow mouth has an exhalation valve, preferably a valve for maintaining the positive end-expiratory pressure, so-called PEEP valve (Positive End-Expiratory Pressure).

Such ventilation systems are widely used in hospitals and homes, especially in the recent periods when their effectiveness has been observed in the treatment of patients with COVID-19 infection.

It has been observed that both the venturi-effect oxygen-air mixer and the exhalation valve constitute sound sources that generate a sound (hiss or puff) at high frequencies, and especially when there are several patients being treated in the same room, can be annoying and decreases comfort for both the patient and the staff in charge of patient care.

A felt need, especially with the latest pandemic spread, for which hospital wards for COVID patients have been set up in which there is a large number of patients being simultaneously treated with continuous forced ventilation, is to make as much tolerable (to hearing) as possible such ventilation devices, both towards the surrounding environment and towards the patient interface.

An object of the present invention is to satisfy these needs left unresolved by known apparatuses and systems, within the framework of a simple, rational and low cost solution.

Such objects are achieved by the characteristics of the invention which are reported in the independent claim. The dependent claims outline preferred and/or particularly advantageous aspects of the invention.

### DISCLOSURE OF THE INVENTION

The invention, particularly, makes available an apparatus for forced ventilation of patients which comprises:
- a silencer body delimiting an internal cavity and provided with at least one access opening that puts the outside of the silencer body in communication with the internal cavity thereof and at least one outlet opening that puts the internal cavity in communication with the outside of the body silencer; wherein the access opening and the outlet opening are separate from each other;
- at least one ventilation device selected from the arrangement consisting of a venturi-effect oxygen-air mixer and an exhalation valve, wherein the ventilation device is at least partially arranged inside the internal cavity of the silencer body and fixed to the silencer body.

Thanks to this solution, a reduction in noise due to the ventilation devices was found both from the point of view of the sound power and in an even more significant and effective way as to the tolerability of the noise from the point of view of the frequency of the emitted sound.

In practice, the silencer body is configured to decrease the frequency of the sound waves emitted by the ventilation device, (as well as to lower the sound power of the sound emitted by the ventilation device, for example by 5 - 15 dB, preferably by 10dB). Preferably, the silencer body can be formed by two half-shells joined together sealingly, for example by bonding.

According to an aspect of the invention, the ventilation device can be rigidly and irremovably fixed to the silencer body.

In this way, the apparatus is of the stand-alone type and ready for use.

Advantageously, the ventilation device can be constituted by the venturi-effect oxygen-air mixer provided with an inlet fitting configured to be connected to an oxygen source, an intake opening configured to suck in air from the outside of the silencer body and an outlet fitting adapted to be connected to an inflow mouth of a patient interface for the delivery of an oxygen-air mixture to the patient.

In this case, wherein the silencer body can comprise:
- at least one housing seat located inside the internal cavity configured to rigidly fix the venturi-effect oxygen-air mixer to the silencer body, and (preferably)
- at least one service opening which puts the internal cavity in communication with the outside of the silencer body;
wherein the access opening of the silencer body is connected sealingly to the inlet fitting of the venturi-effect oxygen-air mixer, the outlet opening of the silencer body is sealingly connected to the outlet fitting of the venturi-effect oxygen-air mixer and the service opening of the silencer body is connected to the intake opening of the venturi-effect oxygen-air mixer.

Thanks to this solution, the air sucked in by venturi effect enters the silencer body and, this effectively reduces the sound impact (both in terms of frequency and in terms of sound power) due to this airflow.

According to an aspect of the invention, the silencer body can comprise a connection path placed inside the internal cavity and configured to connect the service opening of the silencer body to the intake opening of the venturi-effect oxygen-air mixer.

Advantageously, the apparatus can comprise a filter rigidly fixed to the service opening, preferably irremovably.

Thanks to this solution, the reduction of noise and/or sound due to the flow of air sucked in by the Venturi effect is made more effective.

According to a further aspect of the invention, the silencer body can comprise a further access opening which puts the outside of the silencer body in communication with the internal cavity thereof and the venturi-effect oxygen-air mixer is provided with a further inlet fitting configured to be connected to an oxygen source, in this case the apparatus comprises a connection conduit placed inside the internal cavity and configured to connect the further access opening of the silencer body to the further inlet fitting of the venturi-effect oxygen-air mixer.

Thanks to this solution, it is possible to add a greater quantity of oxygen to the oxygen-air mixture obtained by venturi effect from the venturi-effect oxygen-air mixer.

For example, the apparatus may comprise a closure plug removably connected to the further access opening and configured to selectively close and open the further access opening.

According to a further aspect of the invention, the ventilation device can be constituted by the exhalation valve, which comprises a valve body provided with an inlet fitting configured to be connected to a patient interface, at least one vent opening towards the outside of the silencer body and a movable shutter inside the valve body, in which at least the inlet fitting is placed inside the internal cavity of the silencer body.

In this case, the silencer body is configured to reduce the noise caused by the flow of gas passing through the exhalation valve.

Advantageously, the access opening of the silencer body can be configured to be sealingly connected to an outflow mouth of the patient interface, the outlet opening of the silencer body is sealingly connected to the inlet fitting of the exhalation valve.

Furthermore, the silencer body can comprise an accessory opening configured to put the internal cavity in communication with the outside of the silencer body, the apparatus, in turn, comprising an overpressure valve rigidly connected to the service opening.

According to an aspect of the invention, the internal volume (empty or filled with a sound-absorbing/filtering material) of the internal cavity of the silencer body is comprised between 350 cm³ and 450 cm³, for example equal to 400 cm³.

For the same purposes set out above, a further aspect of the invention makes available a forced ventilation system for patients which comprises:
- at least one patient interface configured to deliver a mixture of oxygen and air to the patient and provided with an inflow mouth for the entry of a mixture of oxygen and air and an outflow mouth for the escape of the gases exhaled by the patient;
- at least one apparatus as described above, in which at least one of the access opening and the outlet opening of the silencer body is connected, respectively, to at least one of the outflow mouth and the inflow mouth of the patient interface.

Advantageously, the system can comprise two of said apparatuses, of which
- a first apparatus, in which the ventilation device at least partially contained in the first apparatus is constituted by the venturi-effect oxygen-air mixer connected to the inflow mouth of the patient interface; and
- a second apparatus, in which the ventilation device at least partially contained in the second apparatus is constituted by the exhalation valve connected to the outflow mouth of the patient interface.

Preferably, then, the first apparatus comprises releasable support means adapted to support the second apparatus.

Thanks to this solution, the arrangement of the system is particularly rational, advantageous and convenient for the service staff, as well as distant from the patient and, therefore, more efficient in reducing the noise/sound perceived by the patient.

### BRIEF DESCRIPTION OF THE DRAWINGS

Further features and advantages of the invention will be more apparent after reading the following description provided by way of non-limiting example, with the aid of the accompanying drawings.

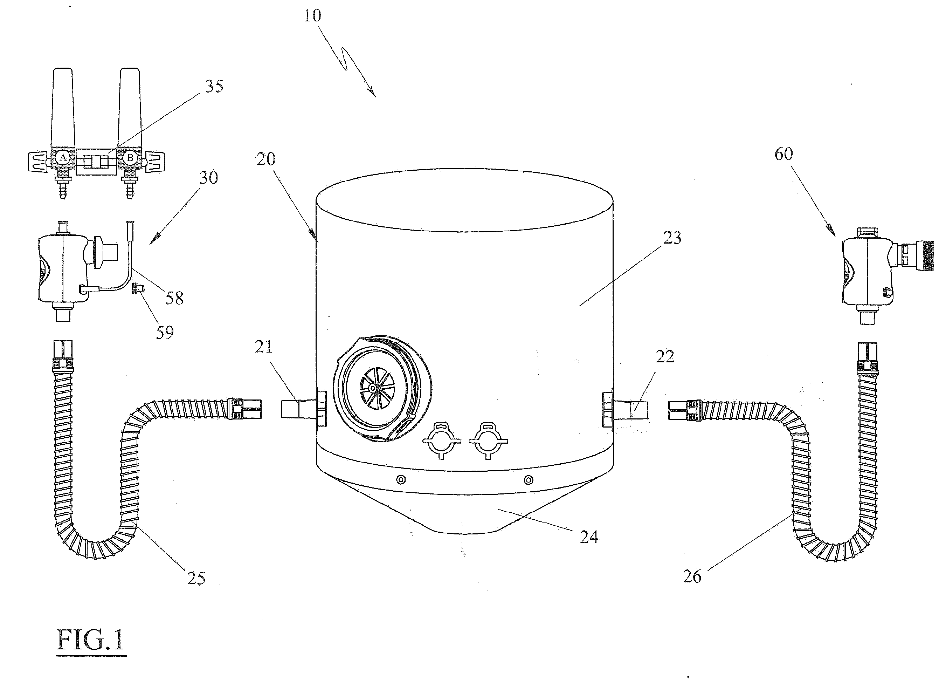

Figure 1 is a schematic view (partially exploded) of a forced ventilation system for patients, according to the invention.

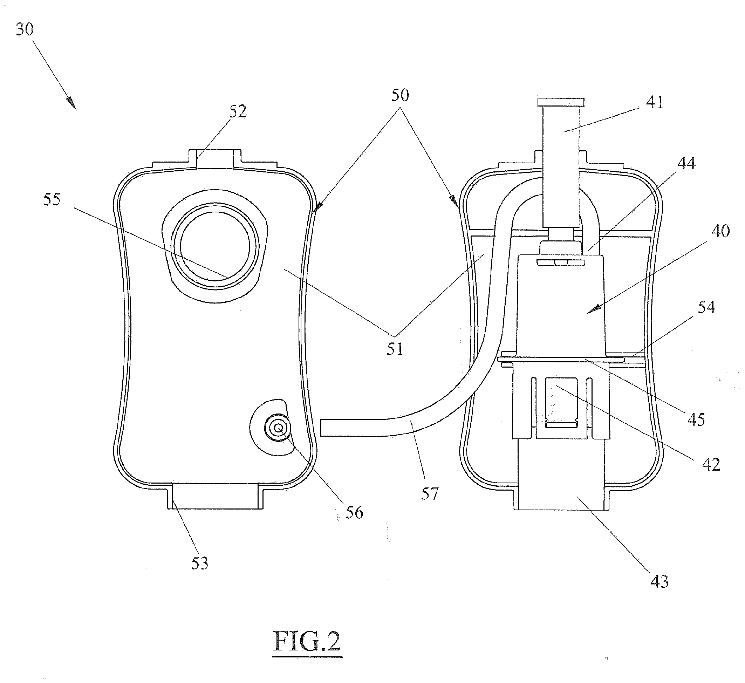

Figure 2 is a view of a first apparatus (open to improve the visibility of the internal components), according to the invention.

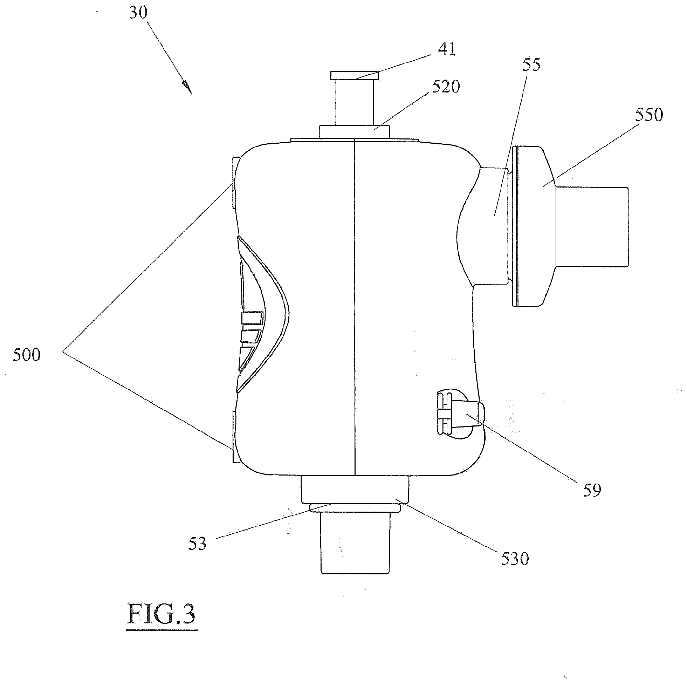

Figure 3 is a side view of the first apparatus of Figure 2, in an assembled configuration.

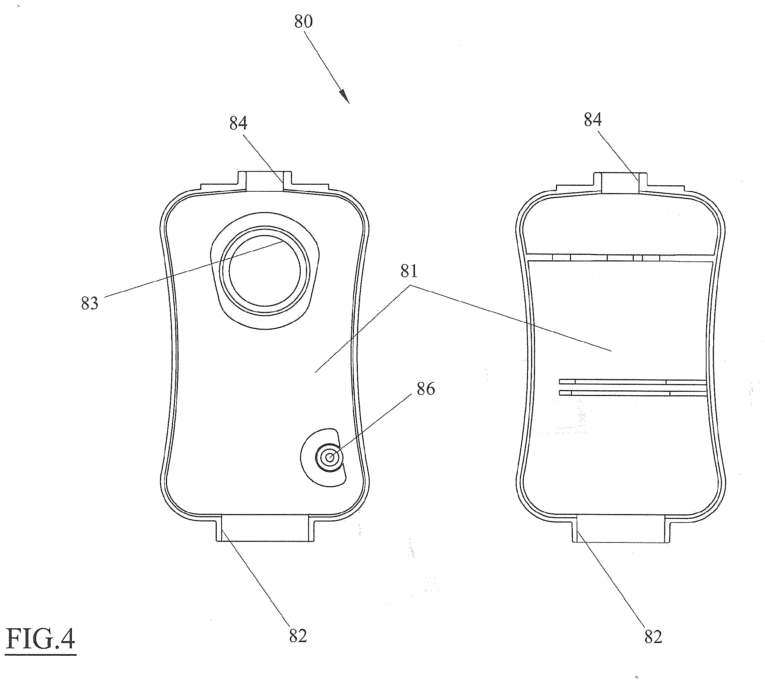

Figure 4 is a view of a second silencer body (open to improve the visibility of the internal components), according to the invention.

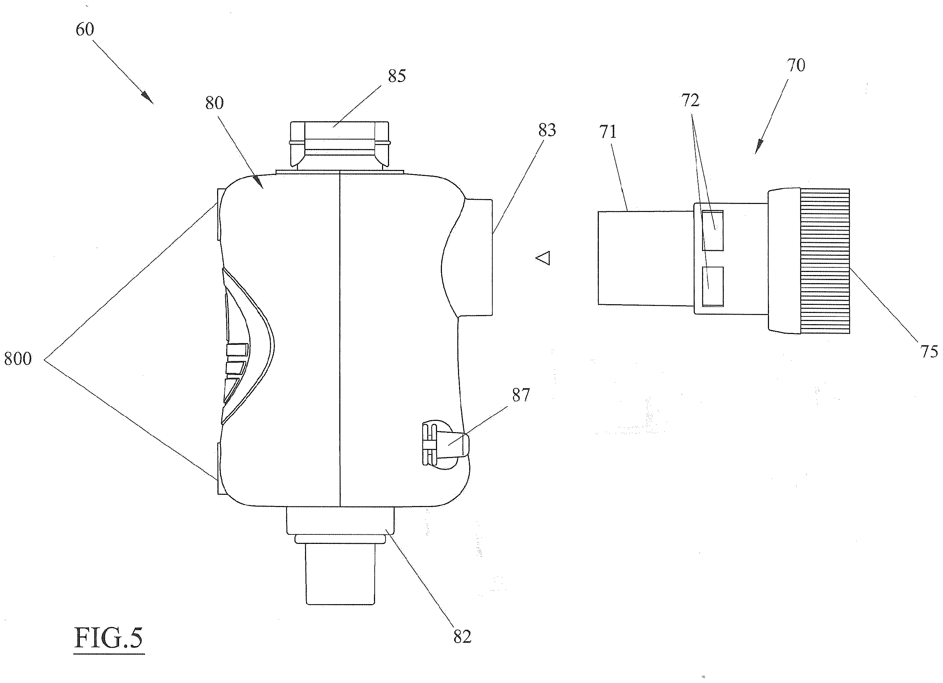

Figure 5 is a side view of a second apparatus, in an assembled configuration.

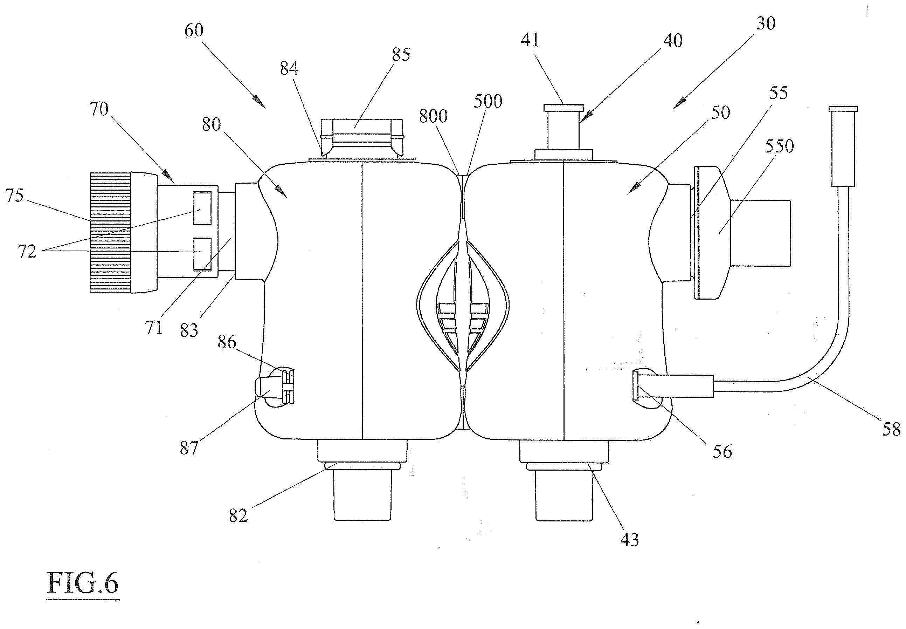

Figure 6 is a side view of a first apparatus and a second apparatus assembled together.

### BEST MODE OF THE INVENTION

With particular reference to these figures, the numeral 10 generally indicates a forced ventilation system for patients, for example for CPAP or NIV therapy.

The system 10 comprises, for example, a patient interface 20 adapted to enclose a volume in communication with at least the mouth and nose of a patient.

The patient interface 20 may further comprise means for introducing a breathable gas, for example a mixture of air and oxygen or pure oxygen or pure air into the volume enclosed by the patient interface 20 and means for the outflow of the gases exhaled by the patient towards the outside of said volume.

In particular, the introduction means comprise an inflow mouth 21 for introducing air (for example provided with a standard-sized fitting for connection to usual pipes for supplying breathable gas, which can be provided, at the end that opens into the volume enclosed by the patient interface, with a diffuser).

The outflow means comprise an outflow mouth 22 for the gases exhaled by the patient during breathing (for example provided with a standard-sized fitting for connection to usual pipes for supplying breathable gas, which can be provided, at the end that opens into the volume enclosed by the patient interface, with a diffuser).

Preferably, the patient interface 20 is provided with an anti-suffocation valve, for example of a known type.

In a first embodiment of the invention shown in the figures, the patient interface 20 comprises (or consists of) a helmet in which the head of a patient can be housed.

In particular, the helmet comprises a substantially cylindrical container body 23, provided with a closed upper end and an opposed open lower end.

The helmet is, for example, made of an optically transparent material which, albeit being flexible, is not expandable.

The helmet further comprises an elastically yielding collar 24, which can be coupled sealingly to the patient's neck and adapted to close the open end of the container body 23.

The collar 24 is advantageously made of elastically yielding material to be coupled sealingly to the patient's neck.

In practice, the collar 24 has a substantially truncated-conical shape, and is provided with a first (widened) end and a second (tapered) open end.

In particular, the first end of the collar 24 has a diameter substantially equal to the diameter of the lower open end of the container body 23 to which said collar 24 is (in a removable or permanent way) fixed, while the second end, of smaller dimensions with respect to the first end, has a diameter comparable or slightly smaller than the diameter of the patient's neck.

In some possible embodiments, the helmet can be for example of the openable type.

In alternative embodiments, the helmet can be of the closed type.

In the helmets of the closed type, the container body 23 is closed by the collar 24 at its lower open end.

In a first variant of the closed type helmet, the collar 24 (i.e. its first end) can be fixed directly to the container body 23 by means of fixing techniques such as heat-sealing or other techniques. For example, in this case the helmet is without any rigid structural parts (such as rings or other that would prevent it from being folded away in narrow spaces).

In a second variant of the helmet of the closed type, between the collar 24 (i.e. the first end thereof) and the container body 23 (i.e. the lower open end thereof) a rigid ring can be interposed, which for example maintains the circular shape of the helmet also when it is not being used.

In the helmets of the openable type, the container body 23 is connected, at its lower open end, to a first rigid ring by means of heat-sealing or other fixing technique which guarantees the hermetic and stable seal between the two.

The collar 24 (i.e. its first end) is connected to a second rigid ring, for example by means of fixing techniques which ensure the hermetic seal between the collar 24 and the second rigid ring thereof.

The second rigid ring, which for example can be coated with soft material, can be sealingly associated, so as to be able to be removed, with the first rigid ring (for example it can be coated with soft material, too), as it will appear better in the following description. The helmet can further comprise an annular gasket comprised among the rigid rings when the helmet is in the closed assembly configuration.

The helmet also comprises releasable locking means adapted to mutually lock in a removable way the first rigid ring and the second rigid ring, which, for example, are selected from the arrangement among bayonet couplings, snap couplings and threaded couplings.

In particular, the rigid rings are provided with locking means of the type of bayonet couplings which comprise two coupling elements which are associated, respectively, to the first rigid ring and to the second rigid ring.

The coupling elements comprise a plurality of pins, for example fixed to a side surface of the second rigid ring and projecting radially externally therefrom.

The coupling elements further comprise a plurality of housing seats, for example obtained in the first rigid ring, spaced from each other so as to accommodate the pins of the second rigid ring.

In practice, each pin is adapted to be inserted in a removable way in a housing seat following a contained mutual translation-rotation between the first rigid ring and the second rigid ring.

In greater detail, the second rigid ring is adapted to be inserted substantially to size inside the first rigid ring, coaxially.

The inflow mouth 21 and/or the outflow mouth 22 can be defined (fixed) at the container body 23, for example diametrically opposite, preferably but not limitedly having an axis directed in a radial direction with respect to a central vertical axis thereof.

It is not excluded, however, that the inflow mouth 21 and/or the outflow mouth 22 can be defined (fixed) at another element constituting the helmet, for example the rigid ring R (in the case of a helmet of the closed type) or the second rigid ring and/or the first rigid ring (in the case of a helmet of the openable type).

Some embodiments of the helmet can envisage that the container body 23 is provided with openings that can be closed by means of a hinge, a porthole (or other) to provide access to the patient.

In a second embodiment of the invention, not shown, the patient interface comprises (or consists of) a mask, for example a mask of the "full-face" type, that is, it can be coupled to the patient's face, in order to inscribe the patient's mouth, nose and eyes.

It is not excluded that the mask may be of the oro-nasal type, that is, it can only surround the patient's nose and mouth (or only the nose or only the mouth).

For example, the mask can be of the rigid or semi-rigid type, i.e. be shaped according to its own shape, for example not modifiable as a result of the operating pressure to which the mask is subjected.

Furthermore, it is not excluded that the mask may be made, for example, without rigid structural parts which determine overall a predefined non-deformable or substantially non-deformable shape.

In this case, the mask can be, for example, an inflatable mask, meaning by inflatable that can alternately pass from a first deflated configuration to a different inflated configuration, in which in the inflated configuration the volume of the mask is different, preferably greater, than the mask volume in the deflated configuration.

The mask, in any case, comprises a container body, also called shell, substantially concave with concavity facing towards the patient's face.

The container body therefore has a concave internal side and an opposed convex external side.

The container body is, for example, made of an optically transparent material. If the mask is a rigid or semi-rigid mask, the container body could be made of a substantially rigid transparent polymeric material, for example polycarbonate (PC) or other material, such as copolyester (PETG). If the mask is an "inflatable" type mask, the container body can be made of a substantially flexible transparent polymeric material, which, albeit being flexible, is not expandable, for example as described in patent no. EP3558433 by the same Applicant.

In practice, said container body protrudes outwards, from the opposite side with respect to the patient's face, inscribing the patient's nose and mouth (and eyes).

The container body could have a shaped portion which protrudes from the frontal surface of the container body towards the outside, approximately in the centre thereof, which is configured to substantially correspond to the patient's nose and/or mouth with mask worn.

This shaped portion has a substantially conical /truncated conical shape.

The container body is advantageously circumscribed by a perimeter edge, which perimeter edge has a substantially trapezoidal shape (with rounded edges), for example of an isosceles trapezoid, with the smaller base placed below in use (in the chin area) and the larger base placed above (in the forehead area).

In practice, the smaller base of the perimeter edge is adapted to rest (even if not directly) on the patient's chin (below the mouth), the larger base is adapted to rest (even if not directly) on the patient's forehead (above the eyes), and the two sides joining the smaller base to the larger base are adapted to rest (even if not directly) on the side of the face, i.e. on the eye and cheek contour (next to the eyes, nose and mouth) .

The mask, therefore, when resting on the patient's face, delimits a closed volume communicating, through the patient's mouth and nose, with the patient's respiratory system. In particular, the mask comprises for example an annular gasket.

In practice, the annular gasket contours the entire perimeter of the perimeter edge of the mask and defines the end portion thereof adapted to come into contact with the patient's face.

The annular gasket could be defined as a single body (for example made by overmould-ing) with the container body, i.e. with the perimeter edge thereof, or be fixed thereto (permanently or in a removable way) as is known to the person skilled in the art. Furthermore, the annular gasket could be of the inflatable type or be made from a solid or pre-inflated frame, as is known in the art.

For example, the annular gasket is made of an elastomeric material (deformable in an elastic or elastic-plastic way), for example in TPE or silicone or other.

The inflow mouth and/or the outflow mouth can be defined (fixed) at the container body, for example coinciding in a same conduit and/or deriving from the same area of the container body or deriving from separate/distinct areas of the container body, for example symmetrically arranged with respect to a sagittal median plane of the mask, preferably but not limitedly having an axis ideally incident on the side of the concavity of the container body.

The system 10 particularly comprises at least one apparatus 30,60 for forced ventilation of patients, which can be used in combination with the patient interface 20 for administering respiratory therapy to the patient.

In particular, the system 10 comprises a first apparatus 30 connected, as will be better described below, to the inflow mouth 21 of the patient interface 20, for the delivery and introduction of a breathable gas (for example an oxygen-air mixture or pure oxygen) inside the volume enclosed by the patient interface 20.

The first apparatus 30 comprises a first ventilation device 40, which is constituted by a venturi-effect oxygen-air mixer, also called a venturi meter.

The first ventilation device 40 comprises, for example, a tubular casing delimiting an internal chamber.

The tubular casing develops along a longitudinal axis, for example in use arranged vertically.

The tubular casing has, substantially in an axially intermediate zone between the opposite axial ends, a venturi groove (or necking), which defines a necking of the internal section of the internal chamber.

At one end (upstream) of the tubular casing there is an (upper) inlet fitting 41, for example of standard dimensions, which is configured to be connected to an oxygen source. The inlet fitting 41 is for example coaxial to the tubular casing.

The tubular casing, moreover, comprises an (lateral) intake opening 42, for example substantially radial, which is configured to suck in air from the outside of the tubular casing towards the inside thereof by Venturi effect.

The intake opening is, for example, arranged at the venturi groove, for example immediately downstream thereof.

At one end (downstream) of the tubular casing, opposite the end provided with the inlet fitting 41, there is an (lower) outlet fitting 43 adapted to be connected (directly or indirectly) to the inflow mouth 21 of a patient interface 20 for the delivery of an oxygen-air mixture to the patient.

The flow of oxygen that crosses the tubular casing from the inlet fitting 41, by venturi effect, draws a flow of air (in a predetermined or predeterminable proportion with respect to the oxygen flow) from the intake opening 42 generating an oxygen-air mixture (that is air enriched with oxygen) that flows in the tubular casing towards the outlet fitting 43 (and from here to the patient interface 20).

The tubular casing can be made in a single body or consist of several parts assembled together.

The area of the intake opening 42 can be fixed, allowing a composition of the oxygen-air mixture that is always constant in proportion, or it can be variable (from a zero value to a maximum value), allowing to adjust the composition of the oxygen-air mixture.

The tubular casing, moreover, may comprise a further (upper) inlet fitting 44 configured to be connected to a source of oxygen.

The further inlet fitting 44 is for example connected, inside the tubular casing, directly to the outlet fitting 43 and/or downstream of the venturi groove.

The further inlet fitting 44 is, in practice, configured to impart to the oxygen-air mixture flowing in the tubular casing towards the outlet fitting 43 a further additional quantity of (a flow of) oxygen, so as to further vary (enrich with oxygen) the composition of the oxygen-air mixture and/or increase the flow of breathable gas leaving the outlet fitting 43.

The tubular casing comprises, externally, a retaining element 45, defined in the example shown by an annular body projecting radially outwards from a central portion of the tubular casing thereof.

The first apparatus 30 comprises, in particular, a first silencer body 50, which is configured to attenuate (in frequency and/or power) the sound/noise caused by the gas flows passing through the first ventilation device 40 (i.e. the venturi-effect oxygen-air mixer). The first silencer body 50 is generally a hollow (thin-walled) body that delimits an internal cavity 51, for example substantially empty or at least partially filled with a sound-absorbing material.

The first silencer body 50 has a substantially cylindrical shape around a central axis (for example, in use, placed vertically), in the example with a circular axial section, closed at the opposite ends by respective closing (dome-like) walls.

Preferably, but not limitedly, the cylindrical shape of the first silencer body 50 is substantially flattened, along a concave curve that generates a central tapered area and two opposing widened ends (like a saddle).

It is not excluded that the first silencer body 50 may have a prismatic shape, with a polygonal section, or have another convenient shape.

The first silencer body 50 is configured to contain within its internal cavity 51 at least a portion of the first ventilation device 40 (that is of the venturi-effect oxygen-air mixer), as will be better described below.

In the illustrated example, the first ventilation device 40 (that is of the venturi-effect oxygen-air mixer) is contained (almost) totally inside the internal cavity 51 of the first silencer body 50, for example with only one (free) end portion of the inlet fitting 41 protruding outside of the first silencer body 50.

The first silencer body 50 comprises an (upper) access opening 52, which is for example located at an axial end of the first silencer body 50, for example centred on the respective closing wall.

The access opening 52 is for example axial, i.e. it is defined by a through hole in the (thin) wall which defines the first silencer body 50 with a through axis parallel (and coincident) to the central axis of the silencer body 50.

The access opening 52 is configured to be sealingly connected to the inlet fitting 41 of the first ventilation device 40 (that is of the venturi-effect oxygen-air mixer).

Preferably, but not limitedly, the access opening 52 is adapted to be fitted (to size) sealingly on the inlet fitting 41 of the first ventilation device 40 (that is of the venturi-effect oxygen-air mixer), so that one of its (free) end portion protrudes outside the internal cavity 51 of the first silencer body 50.

The first silencer body 50 comprises a sealing ring 520 (sealant) adapted to be interposed between the inlet fitting 41 and the access opening 52.

It is not excluded that, before use, the access opening 52 may be closed by a removable plug.

For example, the access opening 52 is adapted to be fitted (to size) sealingly onto the inlet fitting 41 of the first ventilation device 40 (that is of the venturi-effect oxygen-air mixer) in an irremovable manner, for example by means of a sealant adhesive (which defines the aforementioned seal ring 520 and/or fixes it in position).

The first silencer body 50 comprises an outlet opening 53 (lower), which is for example located at an axial end of the silencer body 50, preferably the axial end opposite to that affected by the access opening 52, for example centred on the respective closing wall.

The outlet opening 53 is for example axial, i.e. it is defined by a through hole in the (thin) wall which defines the first silencer body 50 with a through axis parallel (and coincident) to the central axis of the silencer body 50.

For example, the outlet opening 53 has a larger diameter than the access opening 52. The outlet opening 53 is configured to be sealingly connected to the outlet fitting 43 of the first ventilation device 40 (that is of the venturi-effect oxygen-air mixer).

Preferably, but not limitedly, the outlet opening 53 is adapted to be fitted (to size) sealingly on the outlet fitting 43 (for example on the axial end thereof) of the first ventilation device 40 (that is of the venturi-effect oxygen-air mixer).

The first silencer body 50 comprises a further sealing ring 530 (sealant) adapted to be interposed between the outlet fitting 43 and the outlet opening 53.

For example, the outlet opening 53 is adapted to be fitted (to size) sealingly on the outlet fitting 43 of the ventilation device 30 (that is of the venturi-effect oxygen-air mixer) in an irremovable manner, for example by means of a sealant adhesive (which defines the aforementioned seal ring 530 and/or fixes it in position).

The first silencer body 50 comprises at least one housing seat 54 located inside the internal cavity 51 configured to rigidly fix the ventilation device 30, that is the venturi-effect oxygen-air mixer, to the silencer body 50.

For example, the housing seat 54 is configured to hold and fix (in position) the tubular body of the first ventilation device 40 inside the internal cavity 51 of the first silencer body 50.

Preferably, the housing seat 54 is defined by at least one bracket, for example, a double bracket, which axially embraces/contacts the retaining element 45 of the first ventilation device 40.

Furthermore, the housing seat 54 can have a further bracket which embraces an axial portion of the inlet fitting 41 of the first ventilation device 40.

The housing seat 54 can define an interlocking, for example by snap-fit, for the first ventilation device 40.

The first silencer body 50 comprises a (lateral) service opening 55 which puts the internal cavity 51 in (fluidic) communication with the outside of the first silencer body 50. The service opening 55 is open to the outside.

For example, the service opening 55 is located at an intermediate zone between the axial ends of the first silencer body 50, for example proximal to the access opening 52. The service opening 55 is for example radial, i.e. it is defined by a through hole in the (thin) wall which defines the first silencer body 50 with a through axis orthogonal (and incident) to the central axis of the first silencer body 50.

The service opening 55 is surrounded by a cylindrical fitting, for example of standard dimensions.

For example, the service opening 55 has a larger diameter than the access opening 52. The service opening 55 of the first silencer body 50 is connected to the intake opening 42 of the first ventilation device 40, that is of the venturi-effect oxygen-air mixer, for example through the internal cavity 51 of the first silencer body 50.

In practice, between the internal wall of the first silencer body 50 and the external wall of the (tubular body that defines the) first ventilation device 40 a gap is defined (portion of the internal cavity 51), which in turn defines a connection path which puts the service opening 55 of the first silencer body 50 in fluidic communication with the intake opening 42 of the first ventilation device 40.

The (empty) volume of the gap defined in the internal cavity 51 of the first silencer body 50 is substantially comprised between 300 cm³ and 400 cm³, for example equal to 350 cm³.

In practice, this connection path is such as to allow the passage of a forced air flow which enters the service opening 55 drawn by the depression in the internal cavity 51, the depression of which is generated by Venturi effect by the first ventilation 40 by means of its intake opening 42.

The first apparatus 30 preferably comprises a sound suppression element, adapted to at least partially intercept the service opening 55.

For example, the sound suppression element is configured to muffle and/or reduce (in terms of frequency and/or sound power) the sound of the air flow sucked in by the first ventilation device (and which passes through the service opening 55).

Preferably, but not limitedly, the sound suppression element comprises or consists of a filter 550, for example a HEPA filter, which is rigidly fixed (preferably irremovably, for example by bonding) to the service opening 55.

For example, the filter 550 comprises a filter body provided with an access fitting and an outlet fitting, in which the outlet fitting is connected to the service opening 55, for example in a removable way or, more preferably, in an irremovable way.

Inside the filter body there is a filtering septum or filtering membrane, which is adapted to intercept the entire flow of air that passes through the filter.

This filtering septum, for example made of non-woven fabric, is configured to decrease the turbulence of the air entering through the service opening 55 and/or the vibration of the service opening.

The first silencer body 50 also comprises a further (lateral) access opening 56 which puts the outside of the silencer body 50 in communication with the internal cavity 21 thereof.

For example, the further access opening 56 is located at an intermediate zone between the axial ends of the first silencer body 50, for example proximal to the outlet opening 53.

Preferably, but not limitedly, the further access opening 56 faces the same side as the first silencer body 50 with respect to the service opening 55.

The further access opening 56 is for example tangential (or radial), i.e. it is defined by a through hole in the (thin) wall which defines the first silencer body 50 with a through axis orthogonal to the central axis of the first silencer body 50 tangential with respect thereto (i.e. tangential with respect to the cylindrical wall which defines the first silencer body 50.

The further access opening 56 is surrounded by an external cylindrical fitting (which extends externally to the first silencer body 50), for example of standard dimensions and, preferably, also by an internal cylindrical fitting (which extends internally to the internal cavity 51 of the first silencer body 50).

For example, the further access opening 56 has a diameter substantially equal to the diameter of the access opening 52 (or, more precisely, equal to the diameter of the inlet fitting 41 of the first ventilation device 40).

The further access opening 56 of the first silencer body 50 is connected in a fluidic way (and sealingly) to the further inlet fitting 44 of the first ventilation device 40, that is of the venturi-effect oxygen-air mixer.

In practice, the further access opening 56 lengthens the further inlet fitting 44 of the first ventilation device 40 to the outside of the first silencer body 50.

In detail, the first apparatus 30 comprises a connection conduit 57, which is arranged inside the internal cavity 51 of the first container body 50 and is configured to fluidically (and sealingly) interconnect the further access opening 56 with the further inlet fitting 44 of the first ventilation device 40.

The connection conduit 57 is for example a flexible conduit.

Preferably, the connection conduit 57 has a proximal axial end sealingly connected to the (internal cylindrical fitting of) the further access opening 56 and an opposed distal axial end sealingly connected to the further inlet fitting 44 of the first ventilation device 40.

In turn, (the external cylindrical fitting of) the further access opening 56 is connected, for example by means of a further removable connection conduit 58, to a (further) source of oxygen, as will be better described below.

Preferably, the housing seat 54 comprises a compartment suitable for receiving (and fixing in position) at least one axial portion of the connection conduit 57.

The first apparatus 30 further comprises a closure plug 59 which is configured to be removably connected to (the external cylindrical fitting of) the further access opening 56 and configured to selectively close and open the further access opening.

In practice, the closure plug 59 and the further connection conduit 58 can be selectively engaged on (the external cylindrical fitting of) the further access opening 56, according to need.

The silencer body 50 is preferably made by (indissolubly) joining two half-shells, for example separated by a radial separation plane (i.e. parallel and containing the central axis).

These half-shells mate at the perimeter edges and, preferably, are joined together sealingly and in a not openable manner, for example by bonding.

Once the two half-shells are closed, the first ventilation device 40 enclosed therein is, de facto, one piece with the first silencer body 50.

For example, the first silencer body 50 (that is each of the half-shells that compose it) is made of plastic material (which reduces the resonance), for example obtained by injection moulding.

The first apparatus 30 further comprises at least one flow meter 35.

Preferably, the first apparatus 30 comprises a double flow meter 35 (or a pair of flow meters), provided with two oxygen delivery connectors (and at least one oxygen inlet connector, which is connectable to an oxygen source socket, e.g. standard type).

The flow meter 35 is adapted to be supported on the wall, for example through connection to the outlet of the oxygen source.

Preferably, the first silencer body 50 of the first apparatus 30 (and the first ventilation device 40 contained therein) is supported by the flow meter 35.

In other words, the first ventilation device 40 has its own inlet fitting 41 directly connected to (a first oxygen delivery connector of) the flow meter 35 (and the weight of the first silencer body 50 and the first ventilation device 40 contained therein weighs exclusively on the flow meter 35, which supports it suspended).

The further connection conduit 58 can be selectively engaged, at the downstream end thereof, with the further access opening 56 and, at the upstream end thereof, with the second oxygen delivery connector of the flow meter 35.

The first apparatus 30, in particular, the first silencer body 50 thereof, comprises releasable support means placed on the external wall of the first silencer body 50.

The support means, for example, can comprise a magnetic element 500, such as for example a magnet and/or a ferromagnetic element, rigidly fixed to the first silencer body 50.

The support means, alternatively or in addition to the magnetic element 500 can comprise coupling elements, for example hook or snap or bayonet or similar coupling. Preferably, the support means are placed in the silencer body 50 on the opposite side (diametrically opposite) with respect to the service opening 55 and/or to the further access opening 56.

The outlet fitting 43 of the first ventilation device 40 (and/or the outlet opening 53 of the first silencer body 50) is connected (sealingly) to the inflow mouth 21 of the patient interface 20 by means of an inlet pipe 25, for example flexible.

Furthermore, the system 10 can comprise a second apparatus 60 connected, as will be better described below, to the outflow mouth 22 of the patient interface 20, for the escape of the gases exhaled by the patient from the inside of the volume enclosed by the patient interface 20 and control of the patient's end-expiratory pressure.

The second apparatus 60 comprises a second ventilation device 70, which consists of an exhalation valve, preferably a PEEP valve.

The second ventilation device 70, that is the PEEP valve, comprises a tubular valve body, for example substantially cylindrical (and provided with an access conduit 71 (cylindrical and coaxial to the valve body), for example for an axial section with length substantially equal to half the overall length of the second ventilation device 70.

The access conduit 71 defines a connector, for example of standard dimensions (22F or 22M).

The access conduit 71 surrounds an (axial) access mouth in communication with the internal volume of the patient interface 20, which is defined by the aperture of the internal cavity of the access conduit 71.

The valve body of the second ventilation device 70 (that is of the PEEP valve) also comprises an outlet mouth 72, which is spaced along the axis of the valve body from the access mouth defined by the access conduit, as will be better described below.

The second ventilation device 70 also comprises a shutter body (not visible in the figures, but of a known type), for example discoidal, which is placed inside the valve body and is movably associated, for example in translation, from a closed position, in which it occludes the access conduit 71, to an open position, in which it releases it, in contrast to thrust means (which exert a thrust force such as to push the shutter body towards its position closing time).

The thrust means, for example, comprise an elastic element.

The elastic element can comprise a spring (not visible in the figures, but of a known type), in the example a compression spring, for example of the helical type.

In practice, the valve body comprises an internal narrowing, for example annular, defining the valve seat of the shutter body, which is for example discoidal and is movable between a position of contact with the edge of the internal narrowing, in which the second ventilation 70 is closed, and a position of detachment therefrom, in which the second ventilation device 70 is open and allows the gases breathed by the patient to exit from the access conduit 71 to the outlet mouth 72.

An abutment wall 75, which is for example substantially adapted to axially close the valve body, is axially associated with the free end of the valve body, opposite to the access conduit 71.

In the illustrated example, the abutment wall 75 is defined by a (internally) threaded plug, which screws onto a (externally) threaded portion of the free end of the valve body.

The abutment wall 75, therefore, is adapted to be moved by an operator (following screwing/unscrewing), to vary its position with respect to the free (external) end of the valve body.

The spring is for example a compression spring, which is interposed between the shutter body and the abutment wall 75.

By varying the axial position of the abutment wall 75 it is possible to vary the preload of the spring and therefore the resistance exerted by it upon opening the shutter body, between a predetermined first resistance value r1 and a predetermined second resistance value r2, in which the second value r2 which corresponds to a more compressed position of the spring, is greater than the first value r1.

The first resistance value r1 (defined by the minimum compression of the spring, i.e. the maximum distance between the abutment wall 75 and the shutter body) corresponds to a first value of the end-expiratory pressure p1 of the gases inside the internal volume of the patient interface 20 and the second resistance value r2 (defined by the maximum compression of the spring, i.e. the minimum distance between the abutment wall 75 and the shutter body) corresponds to a second end-expiratory pressure value p2 (greater than p1) of the gases within the internal volume of the patient interface 20.

An increase in pressure inside the internal volume of the patient interface 20 (due to the exhalation of the patient under therapy), such as to overcome the action of the spring (and, for example, can be calibrated according to the resistance imposed by the position of the abutment wall 75, between the first value r1 and the second value r2), is adapted to bring the shutter body from the closed position, in which it is normally as a result of the effect of the spring, to the open position.

The narrowing is placed in an intermediate axial position between the access mouth defined by the access conduit 71 and the outlet mouth 72.

The outlet mouth 72 is (radial and) located on the lateral wall of the valve body, for example in an intermediate section thereof axially interposed between the free external end of the valve body and the narrowing.

However, it is not excluded that the outlet mouth may alternatively be (axial) provided at the external free end of the valve body.

In the illustrated example, the valve body comprises a plurality of (equidistant) outlet mouths 72, each defined for example by a radial window.

The second ventilation device 70 could provide a filter, for example antibacterial/antiviral, placed upstream of the shutter body, for example upstream of the access conduit 71 and/or integrated inside the access conduit 71.

The second apparatus 60 particularly comprises a second silencer body 80, which is structurally substantially identical to the first silencer body 50 of the first apparatus 30. The second silencer body 80 is configured to attenuate (in frequency and/or power) the sound/noise caused by the gas flows passing through the second ventilation device 70 (i.e. the PEEP valve).

The second silencer body 80 is overall a hollow (thin-walled) body that delimits an internal cavity 81, for example substantially empty or at least partially filled with a sound-absorbing and/or filtering material.

The second silencer body 80 has a substantially cylindrical shape around a central axis (for example, in use, placed vertically), in the example with a circular axial section, closed at the opposite ends by respective closing (dome-like) walls.

Preferably, but not limitedly, the cylindrical shape of the second silencer body 80 is substantially flattened, along a concave curve that generates a central tapered area and two opposing widened ends (like a saddle).

It is not excluded that the second silencer body 80 may have a prismatic shape, with a polygonal section, or have another convenient shape.

The second silencer body 80 is configured to contain within its internal cavity 81 at least a portion of the second ventilation device 70 (i.e. at least a portion of the PEEP valve), as will be better described below.

The (empty) volume of the internal cavity 81 of the second silencer body 80 is substantially comprised between 350 cm³ and 450 cm³, for example equal to 400 cm³.

In the example illustrated, the second ventilation device 70 (i.e. the PEEP valve) is only partially contained within the internal cavity 81 of the second silencer body 80, for example (only almost all of) the access conduit 71 is contained inside the internal cavity 81 of the second silencer body 80 (and therewith the shutter body, which is the main cause of the noise generated by the second ventilation device 70).

In practice, the outlet mouth 72 (and the abutment wall 75) of the second ventilation device 70 are preferably arranged externally to the second silencer body 80.

The second silencer body 80 comprises an (lower) access opening 82, which is for example located at an axial end of the second silencer body 80, for example centred on the respective closing wall.

The access opening 82 is for example axial, i.e. it is defined by a through hole in the (thin) wall which defines the second silencer body 80 with a through axis parallel to (and coincident) with the central axis of the second silencer body 80.

The access opening 82 is configured to be sealingly connected to the outflow mouth 22 of the patient interface 20.

For example, the access opening 82 of the second silencer body 80 is connected (sealingly) to the outflow mouth 22 of the patient interface 20 by means of an outlet pipe 26, for example flexible.

It is not excluded that, before use, the access opening 82 may be closed by a removable plug.

The second silencer body 80 comprises an (lateral) outlet opening 83, which puts the internal cavity 81 in (fluidic) communication with the outside of the second silencer body 80.

For example, the outlet opening 83 is located at an intermediate zone between the axial ends of the second silencer body 80, for example distal from the access opening 82. The outlet opening 83 is for example radial, i.e. it is defined by a through hole in the (thin) wall which defines the second silencer body 80 with a through axis orthogonal (and incident) to the central axis of the second silencer body 80.

The outlet opening 83 is surrounded by a cylindrical fitting, for example of standard dimensions.

For example, the outlet opening 83 has a diameter substantially equal to the diameter of the access opening 82.

The second ventilation device 70 is connected, sealingly, to the outlet opening 83 of the second silencer body 80, preferably in an irremovable manner.

In particular, the access conduit 71 of the second ventilation device 70 is (coaxially) inserted (to size and/or by interference) inside (the cylindrical fitting that defines) the outlet opening 83.

For example, the access conduit 71 fixed irremovably to (the cylindrical fitting defining) the outlet opening 83 by bonding.

The outlet mouth 72 (and the abutment wall 75) of the second ventilation device 70 are arranged (axially) outside the outlet opening 83 of the second silencer body 80.

The second silencer body 80 may further comprise a (upper) service opening 84 which puts the internal cavity 81 in (fluidic) communication with the outside of the second silencer body 80.

The service opening 84 is, for example, located at an axial end of the silencer body 50, preferably the axial end opposite to that affected by the access opening 82, for example centred on the respective closing wall.

The service opening 84 is for example axial, i.e. it is defined by a through hole in the (thin) wall which defines the second silencer body 80 with a through axis parallel to (and coincident) with the central axis of the second silencer body 80.

The system 10, that is the second apparatus 60 can also comprise an overpressure valve 85, which is configured to put the internal volume of the patient interface 20 (or, preferably, the internal cavity 81 of the second silencer body 80) in communication with the outside when the pressure of the gases contained in the internal volume are greater than a predetermined maximum pressure value Pmax (predefined and fixed in use), which is preferably greater than the second end-expiratory pressure value set in the second pressure ventilation device 70, i.e. in the PEEP valve.

The overpressure valve 85 comprises a valve body, for example substantially cylindrical (placed with a substantially vertical central axis in use) and provided with an inlet conduit (cylindrical and coaxial to the valve body, or an axial lengthening or an axial portion thereof).

The inlet conduit defines a connector, for example of standard size.

The inlet conduit defines an access aperture adapted to be placed in fluidic communication with the internal volume of the patient interface 20 (through the second silencer body 80), which is substantially defined by the aperture of the internal cavity of the inlet conduit at its mouthpiece end.

The valve body of the overpressure valve 85 further comprises an outlet that is opened outwards (which is therefore located externally to the patient interface 20 and/or to the second silencer body 80), which is spaced along the axis of the valve body from the access aperture defined by the inlet conduit.

The outlet is for example made at/near an outflow end opposed to the mouthpiece end of the valve body.

The outlet can be an axial outlet or alternatively it can be a radial outlet made on the side wall of the valve body.

The overpressure valve 85 also comprises a shutter, for example discoidal, which is placed (coaxially) inside the valve body and is movably associated, for example in translation, from a closed position, in which it occludes the access aperture of the mouthpiece end, to an open position, in which it releases it, in contrast to thrust means, for example of the type of a spring, which is configured to exert a predetermined and fixed force on the shutter.

Therefore, the resistance conveyed by the spring when the shutter opens is equal to a predetermined and fixed maximum resistance value r3 (greater than the predetermined second resistance value r2 of the spring of the PEEP valve).

The maximum resistance value r3 corresponds to the maximum pressure value Pmax allowed for the gases within the internal volume of the patient interface 20.

An increase in pressure inside the internal volume of the helmet 10 (higher than the maximum pressure value Pmax allowed), such as to overcome the action of the spring of the overpressure valve 85, is adapted to bring the shutter thereof from the closed position, in which it is normally as a result of the effect of the spring, to the open position, allowing venting and restoring the correct pressure level in the patient interface 20.

The overpressure valve 85 could provide a filter, for example antibacterial/antiviral, placed upstream of the shutter, for example upstream of the inlet conduit and/or integrated inside the inlet conduit.

The overpressure valve 85 is connected, in a sealed manner, to the service opening 84 of the second silencer body 80, preferably in an irremovable manner.

In particular, the inlet conduit of the overpressure valve 85 is (coaxially) inserted (to size and/or by interference) inside the service opening 84.

For example, the inlet conduit of the overpressure valve 85 is fixed irremovably to the service port 84 by bonding.

The outlet of the overpressure valve 85 is arranged (axially) outside the service opening 84 of the second silencer body 80, so as to be directed (and vent) towards the outside of the second silencer body 80.

The second silencer body 80 can also comprise a further (lateral) service opening 86 which puts the outside of the second silencer body 80 in communication with the internal cavity 81 thereof.

For example, the further service opening 86 is located at an intermediate zone between the axial ends of the second silencer body 80, for example proximal to the access opening 82.

Preferably, but not limitedly, the further service opening 86 faces the same side as the second silencer body 80 with respect to the outlet opening 83.

The further service opening 86 is for example tangential (or radial), i.e. it is defined by a through hole in the (thin) wall which defines the second silencer body 80 with a through axis orthogonal to the central axis of the second silencer body 80 and tangential with respect thereto (i.e. tangential with respect to the cylindrical wall which defines the second silencer body 80).

The further service opening 86 is surrounded by an external cylindrical fitting (which extends externally to the second silencer body 80), for example of standard dimensions and, preferably, also by an internal cylindrical fitting (which extends internally to the internal cavity 81 of the second silencer body 80).

The second apparatus 60 further comprises a closure plug 87 which is configured to be removably connected to (the external cylindrical fitting of) the further service opening 86 and configured to selectively close and open the further inlet opening or close it in an immovable way (for example by bonding).

The second silencer body 80 is preferably made by (indissolubly) joining two half-shells, for example separated by a radial separation plane (i.e. parallel and containing the central axis).

These half-shells mate at the perimeter edges and, preferably, are joined together sealingly and in a not openable manner, for example by bonding.

Once the two half-shells are closed, the second ventilation device 70 fixed therein is, de facto, one piece with the second silencer body 80.

For example, the second silencer body 80 (or each of the half-shells that compose it) is made of plastic material (which reduces the resonance), for example obtained by injection moulding.

The second apparatus 60, in particular the second silencer body 80 thereof, comprises releasable support means placed on the external wall of the second silencer body 80. The support means of the second apparatus 60 are for example configured to be connected to the support means of the first apparatus 30, for example in a releasable way. The support means of the second apparatus 60, for example, are complementary (and connectable so as to define a stable connection) with respect to the support means of the first apparatus 30.

The support means of the second apparatus 60, for example, can comprise a magnetic element 800, such as for example a magnet and/or a ferromagnetic element or a ferrous body, rigidly fixed to the second silencer body 80.

The support means, alternatively or in addition to the magnetic element 800 can comprise coupling elements, for example hook or snap or bayonet or similar coupling. Preferably, the support means are placed in the second silencer body 80 on the opposite side (diametrically opposite) with respect to the outlet opening 83 and/or to the further service opening 85.

In practice, the second silencer body 80 of the second apparatus 60 (and the second ventilation device 70 fixed thereto) is supported (suspended/cantilevered) by the first silencer body 50 through the connection between the support means (of the first silencer body 50 and of the second silencer body 60), for example when the first silencer body 50 is in turn supported by the flow meter 35.

In light of the above, the operation of the system 10 is as follows.

For the respiratory therapy of a patient to be carried out, the connection of the flow meter 35 to the oxygen source is first set up.

At this point, the first apparatus 30 is connected to the flow meter 35, for example by means of the (direct) connection of the inlet fitting 41 to the first oxygen delivery connector of the flow meter 35 and, preferably, the connection of the further access opening 56 (of the first silencer body 50) to the second oxygen delivery connector of the flow meter 35, by means of the further connection conduit 58 (supplied).

The outlet fitting 43 of the first ventilation device 40 (that is of the venturi-effect oxygen-air mixer) and/or the outlet opening 53 of the first silencer body 50 is connected to the inflow mouth 21 of the patient interface 20, preferably via the inlet pipe 25.

At this point, the outflow mouth 22 of the patient interface 20 is connected to the second ventilation device 70, that is to a PEEP valve.

In a simplified version of the system 10, the PEEP valve can be directly connected to the outflow mouth 22 of the patient interface 20 or by means of a suitable outlet pipe 26. In a preferred version of the system 10, the outflow mouth 22 of the patient interface 20 is connected to the second apparatus 60, that is, it is connected to the second ventilation device 70 by means of (interposition of) the second silencer body 80.

In detail, the access opening 82 of the second silencer body 80 is connected to the outflow mouth 22 of the patient interface 20, preferably by interposition of the outlet pipe 26.

The internal volume of the patient interface 20 is therefore connected to the outside by means of the outlet mouth 72 of the second ventilation device 70 (i.e. the PEEP valve) and/or the overpressure valve 85.

The patient interface 20 can be worn by the patient for initiating the respiratory therapy. When the flow of oxygen passes through the first ventilation device 40, the air sucked by it (from the outside) first passes through the filter 550 and then enters through the access opening 52 of the first silencer body 50 into the (gap of the) internal cavity 51 of the first silencer body 50 thereof.

This (gap of the) internal cavity 51 acts as an expansion chamber, which allows to decrease the frequency and/or the sound power of the sound produced by the first ventilation device 40.

The first silencer body 50 essentially encapsulates the first ventilation device 40 reducing its sound impact, i.e. reducing the frequency of the sound produced by the first ventilation device 40 (compared to the same first ventilation device without the first silencer body) and/or by reducing the sound power thereof.

It has been observed that, thanks to the conformation and the presence of the first silencer body 50, the first apparatus 30 emits a sound whose sound power is comprised between 60 dB and 70 dB, for example equal to 65 dB (compared to a sound power 75 dB emitted by any first ventilation device 40 without the first silencer body 50).

During the operation of the system 10, then, the second ventilation device 70 (i.e. the PEEP valve) comes into operation, that is, it opens whenever the pressure inside the patient interface 20 exceeds the set end-exhalation pressure.

The gaseous flow that moves the shutter body of the second ventilation device 70 causes a further sound (for example intermittent), which is muffled and made more tolerable (for example due to the decrease in frequency it undergoes) by the presence of the second silencer body 80.

It has been observed that, thanks to the conformation and the presence of the second silencer body 80, the second apparatus 60 emits a sound whose sound power is comprised between 45 dB and 55 dB, for example equal to 50 dB (compared to a sound power of 55 dB - 75 dB emitted by any second ventilation device 70 without the second silencer body 80).

The first apparatus 30, as described above, is rigidly fixed to and supported by the flow meter 35.

The second apparatus 60 can therefore be associated with the first apparatus by means of the respective support means, for example by connecting the magnetic element 800 of the second silencer body to the magnetic element 500 of the first silencer body.

In this way, during use, the second apparatus 60 is supported and kept in position (remote from the patient interface 20) by the first apparatus 30.

The invention thus conceived is susceptible to several modifications and variations, all falling within the scope of the inventive concept.

Moreover, all the details can be replaced by other technically equivalent elements.

In practice, the materials used, as well as the contingent shapes and sizes, can be whatever according to the requirements without for this reason departing from the scope of protection of the following claims.

## Claims

1. An apparatus (30,60) for forced ventilation of patients which comprises:
- at least one ventilation device (40,70) selected from the arrangement consisting of a venturi-effect oxygen-air mixer and an exhalation valve;
**characterized in that** the apparatus (30,60) comprises:
- a silencer body (50,80) delimiting an internal cavity (51,81) and provided with at least one access opening (52,82) that puts the outside of the silencer body (50,80) in communication with the internal cavity (51,81) thereof and at least one outlet opening (53,83) that puts the internal cavity (51,81) in communication with the outside of the silencer body (50,80); wherein the access opening (52,82) and the outlet opening (53,83) are separate from each other;
**and in that** the ventilation device (40,70) is at least partially arranged inside the internal cavity (51,81) of the silencer body (50,80) and fixed to the silencer body (50,80).

2. The apparatus (30,60) according to claim 1, wherein the silencer body (50,80) is formed by two half-shells joined together sealingly in an unopenable manner.

3. The apparatus (30,60) according to claim 2, wherein the two half-shells are joined together by bonding.

4. The apparatus (30,60) according to claim 1, wherein the ventilation device (40,70) is rigidly and irremovably fixed to the silencer body (50,80).

5. The apparatus (30,60) according to claim 1, wherein the ventilation device (40) is constituted by the venturi-effect oxygen-air mixer provided with an inlet fitting (41) configured to be connected to an oxygen source, an intake opening (42) configured to suck in air from the outside of the silencer body (50) and an outlet fitting (43) adapted to be connected to an inflow mouth (21) of a patient interface (20) for the delivery of an oxygen-air mixture to the patient.

6. The apparatus (30,60) according to the preceding claim, wherein the silencer body (50) comprises:
- at least one housing seat (54) located inside the internal cavity (51) configured to rigidly fix the venturi-effect oxygen-air mixer to the silencer body (50), and
- at least one service opening (55) which puts the internal cavity (51) in communication with the outside of the silencer body (50);
wherein the access opening (52) of the silencer body (50) is connected sealingly to the inlet fitting (41) of the venturi-effect oxygen-air mixer, the outlet opening (53) of the silencer body (50) is sealingly connected to the outlet fitting (43) of the venturi-effect oxygen-air mixer and the service opening (55) of the silencer body (50) is connected to the intake opening (42) of the venturi-effect oxygen-air mixer.

7. The apparatus (30,60) according to the preceding claim, which comprises a connection path located inside the internal cavity (51) and configured to connect the service opening (55) of the silencer body (50) to the intake opening (42) of the venturi-effect oxygen-air mixer.

8. The apparatus (30,60) according to claim 6, which comprises a filter (550) rigidly fixed to the service opening (55).

9. The apparatus (30,60) according to the preceding claim, wherein the filter (550) is irremovably fixed to the service opening (55).

10. The apparatus (30,60) according to claim 6, wherein the silencer body (50) comprises a further access opening (56) which puts the outside of the silencer body (50) in communication with the internal cavity (51) thereof and the venturi-effect oxygen-air mixer is provided with a further inlet fitting (44) configured to be connected to an oxygen source, wherein the apparatus (30,60) comprises a connection conduit (57) placed inside the internal cavity (51) and configured to connect the further access opening (56) of the silencer body (50) to the further inlet fitting (44) of the venturi-effect oxygen-air mixer.

11. The apparatus (30,60) according to the preceding claim, which comprises a closure plug (59) removably connected to the further access opening (56) and configured to selectively close and open the further access opening (56).

12. The apparatus (30,60) according to claim 1, wherein the ventilation device (70) is constituted by the exhalation valve, which comprises a valve body provided with an inlet fitting (71) configured to be connected to a patient interface (20), at least one vent opening (72) towards the outside of the silencer body (80) and a movable shutter inside the valve body, wherein at least the inlet fitting (71) is placed inside the internal cavity (81) of the silencer body (80).

13. The apparatus (30,60) according to the preceding claim, wherein the access opening (82) of the silencer body (80) is configured to be connected to an outflow mouth (22) of the patient interface (20), the outlet opening (83) of the silencer body (80) is sealingly connected to the inlet fitting (71) of the exhalation valve.

14. The apparatus (30,60) according to claim 12, wherein the silencer body (80) comprises a service opening (84) configured to put the internal cavity (81) in communication with the outside of the silencer body (80), the apparatus (30,60) comprising an overpressure valve (85) rigidly connected to the service opening (84).

15. The apparatus (30,60) according to claim 1, wherein the internal volume of the internal cavity (51,81) is comprised between 350 cm³ and 450 cm³.

16. A forced ventilation system (10) for patients that comprises:
- at least one patient interface (20) configured to deliver a mixture of oxygen and air to the patient and provided with an inflow mouth (21) for the entry of a mixture of oxygen and air and an outflow mouth (22) for the escape of the gases exhaled by the patient;
- at least one apparatus (30,60) according to claim 1, wherein at least one of the access opening (52,82) and the outlet opening (53,83) of the silencer body (50,80) is connected, respectively, to at least one of the outflow mouth (22) and the inflow mouth (21) of the patient interface (20).

17. The system (10) according to the preceding claim, which comprises two of said apparatuses (30,60), of which
- a first apparatus (30), in which the ventilation device (40) at least partially contained in the first apparatus is constituted by the venturi-effect oxygen-air mixer connected to the inflow mouth (21) of the patient interface (20); and
- a second apparatus (60), in which the ventilation device (70) at least partially contained in the second apparatus (60) is constituted by the exhalation valve connected to the outflow mouth (22) of the patient interface (20).

18. The system (10) according to the preceding claim, wherein the first apparatus (30) comprises releasable support means (500) adapted to support the second apparatus (60).

## Patentansprüche

1. Gerät (30, 60) zur Zwangsbeatmung von Patienten, das Folgendes umfasst:
- mindestens eine Beatmungsvorrichtung (40, 70), ausgewählt aus der Anordnung bestehend aus einem Venturi-Effekt-Sauerstoff-Luft-Mischer und einem Ausatmungsventil;
**dadurch gekennzeichnet, dass** die Gerät (30, 60) Folgendes umfasst:
- einen Schalldämpferkörper (50, 80), der einen inneren Hohlraum (51, 81) begrenzt und mit mindestens einer Zugangsöffnung (52, 82) versehen ist, die die Außenseite des Schalldämpferkörpers (50, 80) mit dem innerem Hohlraum (51, 81) davon in Verbindung bringt, und mit mindestens einer Auslassöffnung (53, 83), die den inneren Hohlraum (51, 81) mit der Außenseite des Schalldämpferkörpers (50, 80) in Verbindung bringt; wobei die Zugangsöffnung (52, 82) und die Auslassöffnung (53, 83) voneinander getrennt sind;
**und dass** die Beatmungsvorrichtung (40, 70) zumindest teilweise in dem inneren Hohlraum (51, 81) des Schalldämpferkörpers (50, 80) angeordnet und an dem Schalldämpferkörper (50, 80) befestigt ist.

2. Gerät (30, 60) nach Anspruch 1, wobei der Schalldämpferkörper (50, 80) durch zwei Halbschalen gebildet ist, die abdichtend auf eine nicht zu öffnende Weise miteinander verbunden sind.

3. Gerät (30, 60) nach Anspruch 2, wobei die zwei Halbschalen durch Verkleben miteinander verbunden sind.

4. Gerät (30, 60) nach Anspruch 1, wobei die Beatmungsvorrichtung (40, 70) starr und unabnehmbar an dem Schalldämpferkörper (50, 80) befestigt ist.

5. Gerät (30, 60) nach Anspruch 1, wobei die Beatmungsvorrichtung (40) durch den Venturi-Effekt-Sauerstoff-Luft-Mischer gebildet ist, der mit einem Einlassanschluss (41), der konfiguriert ist, um mit einer Sauerstoffquelle verbunden zu werden kann, einer Einlassöffnung (42), die konfiguriert ist, um Luft von der Außenseite des Schalldämpferkörpers (50) anzusaugen, und einem Auslassanschluss (43), der angepasst ist, um mit einer Einlassöffnung (21) einer Patientenschnittstelle (20) für die Abgabe eines Sauerstoff-Luft-Gemischs an den Patienten verbunden zu werden, versehen ist.

6. Gerät (30, 60) nach dem vorherigen Anspruch, wobei der Schalldämpferkörper (50) Folgendes umfasst:
- mindestens einen Gehäusesitz (54), der sich im Inneren des inneren Hohlraums (51) befindet, der konfiguriert ist, um den Venturi-Effekt-Sauerstoff-Luft-Mischer starr an dem Schalldämpferkörper (50) zu befestigen, und
- mindestens eine Serviceöffnung (55), die den inneren Hohlraum (51) mit der Außenseite des Schalldämpferkörpers (50) in Verbindung bringt;
wobei die Zugangsöffnung (52) des Schalldämpferkörpers (50) abdichtend mit dem Einlassanschluss (41) des Venturi-Effekt-Sauerstoff-Luft-Mischers verbunden ist, die Auslassöffnung (53) des Schalldämpferkörpers (50) abdichtend mit dem Auslassanschluss (43) des Venturi-Effekt-Sauerstoff-Luft-Mischers verbunden ist und die Serviceöffnung (55) des Schalldämpferkörpers (50) mit der Einlassöffnung (42) des Venturi-Effekt-Sauerstoff-Luft-Mischers verbunden ist.

7. Gerät (30, 60) nach dem vorherigen Anspruch, das einen Verbindungsweg umfasst, der sich im Inneren des inneren Hohlraums (51) befindet und konfiguriert ist, um die Serviceöffnung (55) des Schalldämpferkörpers (50) mit der Einlassöffnung (42) des Venturi-Effekt-Sauerstoff-Luft-Mischers zu verbinden.

8. Gerät (30, 60) nach Anspruch 6, das einen Filter (550) umfasst, der starr an der Serviceöffnung (55) befestigt ist.

9. Gerät (30, 60) nach dem vorherigen Anspruch, wobei der Filter (550) unabnehmbar an der Serviceöffnung (55) befestigt ist.

10. Gerät (30, 60) nach Anspruch 6, wobei der Schalldämpferkörper (50) eine weitere Zugangsöffnung (56) aufweist, die die Außenseite des Schalldämpferkörpers (50) in Verbindung mit dem inneren Hohlraum (51) davon bringt, und der Venturi-Effekt-Sauerstoff-Luft-Mischer mit einem weiteren Einlassanschluss (44) versehen ist, der konfiguriert ist, um mit einer Sauerstoffquelle verbunden zu werden, wobei das Gerät (30, 60) eine Verbindungsleitung (57) umfasst, die in dem inneren Hohlraum (51) platziert und konfiguriert ist, um die weitere Zugangsöffnung (56) des Schalldämpferkörpers (50) mit dem weiteren Einlassanschluss (44) des Venturi-Effekt-Sauerstoff-Luft-Mischers zu verbinden.

11. Gerät (30, 60) nach dem vorherigen Anspruch, das einen Verschlussstopfen (59) umfasst, der abnehmbar mit der weiteren Zugangsöffnung (56) verbunden und konfiguriert ist, um die weitere Zugangsöffnung (56) selektiv zu schließen und zu öffnen.

12. Gerät (30, 60) nach Anspruch 1, wobei die Beatmungsvorrichtung (70) durch das Ausatmungsventil gebildet ist, das einen Ventilkörper umfasst, der mit einem Einlassanschluss (71), der konfiguriert ist, um mit einer Patientenschnittstelle (20) verbunden zu werden, mindestens einer Entlüftungsöffnung (72) zu der Außenseite des Schalldämpferkörpers (80) und einer beweglichen Klappe innerhalb des Ventilkörpers versehen ist, wobei mindestens der Einlassanschluss (71) innerhalb des inneren Hohlraums (81) des Schalldämpferkörpers (80) platziert ist.

13. Gerät (30, 60) nach dem vorherigen Anspruch, wobei die Zugangsöffnung (82) des Schalldämpferkörpers (80) konfiguriert ist, um mit einer Ausflussmündung (22) der Patientenschnittstelle (20) verbunden zu sein, und die Auslassöffnung (83) des Schalldämpferkörpers (80) abdichtend mit dem Einlassanschluss (71) des Ausatmungsventils verbunden ist.

14. Gerät (30, 60) nach Anspruch 12, wobei der Schalldämpferkörper (80) eine Serviceöffnung (84) umfasst, die konfiguriert ist, um den inneren Hohlraum (81) mit der Außenseite des Schalldämpferkörpers (80) in Verbindung zu bringen, das Gerät (30, 60) umfassend ein Überdruckventil (85) umfasst, das starr mit der Serviceöffnung (84) verbunden ist.

15. Gerät (30, 60) nach Anspruch 1, wobei das Innenvolumen des inneren Hohlraums (51, 81) zwischen 350 cm³ und 450 cm³ liegt.

16. Zwangsbeatmungssystem (10) für Patienten, das Folgendes umfasst:
- mindestens eine Patientenschnittstelle (20), die konfiguriert ist, um dem Patienten ein Gemisch aus Sauerstoff und Luft zuzuführen, und die mit einer Einflussmündung (21) für den Eintritt eines Gemischs aus Sauerstoff und Luft und einer Ausflussmündung (22) für den Austritt der Gase versehen ist, die von dem Patienten ausgeatmet werden;
- mindestens ein Gerät (30, 60) nach Anspruch 1, wobei mindestens eine von der Zugangsöffnung (52, 82) und der Auslassöffnung (53, 83) des Schalldämpferkörpers (50, 80) mit mindestens einer von der Ausflussmündung (22) und der Einflussmündung (21) der Patientenschnittstelle (20) verbunden ist.

17. System (10) nach dem vorherigen Anspruch, das zwei der Geräte (30, 60) umfasst, darunter
- ein erstes Gerät (30), wobei die Beatmungsvorrichtung (40), die zumindest teilweise in dem ersten Gerät enthalten ist, aus dem Venturi-Effekt-Sauerstoff-Luft-Mischer besteht, der mit der Einflussmündung (21) der Patientenschnittstelle (20) verbunden ist; und
- ein zweites Gerät (60), wobei die Beatmungsvorrichtung (70), die zumindest teilweise in dem zweiten Gerät (60) enthalten ist, aus dem Ausatmungsventil besteht, das mit der Ausflussmündung (22) der Patientenschnittstelle (20) verbunden ist.

18. System (10) nach dem vorherigen Anspruch, wobei das erste Gerät (30) lösbare Halterungseinrichtungen (500) umfasst, die angepasst sind, um das zweite Gerät (60) zu halten.

## Revendications

1. Appareil (30,60) destiné à la ventilation forcée des patients comprenant :
- au moins un dispositif de ventilation (40,70) choisi parmi l'ensemble constitué d'un mélangeur oxygène-air à effet venturi et d'un clapet d'expiration ;
**caractérisé en ce que** l'appareil (30,60) comprend:
- un corps de silencieux (50,80) délimitant une cavité interne (51,81) et pourvu d'au moins une ouverture d'accès (52,82) qui met l'extérieur du corps de silencieux (50,80) en communication avec sa cavité interne (51,81), et d'au moins une ouverture de sortie (53,83) qui met la cavité interne (51,81) en communication avec l'extérieur du corps de silencieux (50,80) ; dans lequel l'ouverture d'accès (52,82) et l'ouverture de sortie (53,83) sont séparées l'une de l'autre ;
**et en ce que** le dispositif de ventilation (40,70) est au moins en partie disposé à l'intérieur de la cavité interne (51,81) du corps de silencieux (50,80) et fixé au corps de silencieux (50,80).

2. Appareil (30,60) selon la revendication 1, dans lequel le corps de silencieux (50,80) est formé de deux demi-coques assemblées de manière étanche et non ouvrable.

3. Appareil (30,60) selon la revendication 2, dans lequel les deux demi-coques sont assemblées par collage.

4. Appareil (30,60) selon la revendication 1, dans lequel le dispositif de ventilation (40,70) est fixé de manière rigide et inamovible au corps de silencieux (50,80).

5. Appareil (30,60) selon la revendication 1, dans lequel le dispositif de ventilation (40) est constitué du mélangeur oxygène-air à effet venturi doté d'un raccord d'entrée (41) configuré pour être connecté à une source d'oxygène, d'une ouverture d'admission (42) configurée pour aspirer l'air depuis l'extérieur du corps de silencieux (50) et d'un raccord de sortie (43) adapté pour être connecté à une ouverture d'entrée (21) d'une interface patient (20) pour l'administration d'un mélange oxygène-air au patient.

6. Appareil (30,60) selon la revendication précédente, dans lequel le corps de silencieux (50) comprend :
- au moins un siège de boîtier (54) situé à l'intérieur de la cavité interne (51) configuré pour fixer rigidement le mélangeur oxygène-air à effet venturi au corps de silencieux (50), et
- au moins une ouverture de service (55) mettant la cavité interne (51) en communication avec l'extérieur du corps de silencieux (50) ;
dans lequel l'ouverture d'accès (52) du corps de silencieux (50) est reliée de manière étanche au raccord d'entrée (41) du mélangeur oxygène-air à effet venturi, l'ouverture de sortie (53) du corps de silencieux (50) est reliée de manière étanche au raccord de sortie (43) du mélangeur oxygène-air à effet venturi et l'ouverture de service (55) du corps de silencieux (50) est reliée à l'ouverture d'admission (42) du mélangeur oxygène-air à effet venturi.

7. Appareil (30,60) selon la revendication précédente, comprenant une voie de liaison située à l'intérieur de la cavité interne (51) et configurée pour relier l'ouverture de service (55) du corps de silencieux (50) à l'ouverture d'admission (42) du mélangeur oxygène-air à effet venturi.

8. Appareil (30,60) selon la revendication 6, comprenant un filtre (550) fixé de manière rigide à l'ouverture de service (55).

9. Appareil (30,60) selon la revendication précédente, dans lequel le filtre (550) est fixé de manière inamovible à l'ouverture de service (55).

10. Appareil (30,60) selon la revendication 6, dans lequel le corps de silencieux (50) comprend une autre ouverture d'accès (56) mettant l'extérieur du corps de silencieux (50) en communication avec la cavité interne (51) de celui-ci et le mélangeur oxygène-air à effet venturi est pourvu d'un autre raccord d'entrée (44) configuré pour être connecté à une source d'oxygène, dans lequel l'appareil (30,60) comprend un canal de connexion (57) placé à l'intérieur de la cavité interne (51) et configuré pour connecter l'autre ouverture d'accès (56) du corps de silencieux (50) à l'autre raccord d'entrée (44) du mélangeur oxygène-air à effet venturi.

11. Appareil (30,60) selon la revendication précédente, comprenant un bouchon de fermeture (59) relié de manière amovible à l'autre ouverture d'accès (56) et configuré pour fermer et ouvrir sélectivement l'autre ouverture d'accès (56).

12. Appareil (30,60) selon la revendication 1, dans lequel le dispositif de ventilation (70) est constitué du clapet d'expiration, qui comprend un corps de clapet pourvu d'un raccord d'entrée (71) configuré pour être connecté à une interface patient (20), au moins une ouverture de ventilation (72) vers l'extérieur du corps de silencieux (80) et un obturateur mobile à l'intérieur du corps de clapet, dans lequel au moins le raccord d'entrée (71) est placé à l'intérieur de la cavité interne (81) du corps de silencieux (80).

13. Appareil (30,60) selon la revendication précédente, dans lequel l'ouverture d'accès (82) du corps de silencieux (80) est configurée pour être connectée à une ouverture d'écoulement (22) de l'interface patient (20), l'ouverture de sortie (83) du corps de silencieux (80) étant connectée de manière étanche au raccord d'entrée (71) du clapet d'expiration.

14. Appareil (30,60) selon la revendication 12, dans lequel le corps de silencieux (80) comprend une ouverture de service (84) configurée pour mettre la cavité interne (81) en communication avec l'extérieur du corps de silencieux (80), l'appareil (30,60) comprenant une soupape de surpression (85) rigidement reliée à l'ouverture de service (84).

15. Appareil (30,60) selon la revendication 1, dans lequel le volume interne de la cavité interne (51,81) est compris entre 350 cm³ et 450 cm³.

16. Système de ventilation forcée (10) destiné aux patients et comprenant :
- au moins une interface patient (20) configurée pour délivrer un mélange oxygène-air au patient et dotée d'une ouverture d'entrée (21) pour l'entrée d'un mélange oxygène-air et d'une ouverture d'écoulement (22) pour l'évacuation des gaz expirés par le patient ;
- au moins un appareil (30,60) selon la revendication 1, dans lequel au moins une des ouvertures d'accès (52,82) et de sortie (53,83) du corps de silencieux (50,80)est connectée, respectivement, à au moins une des ouvertures d'écoulement (22) et d'entrée (21) de l'interface patient (20).

17. Système (10) selon la revendication précédente, comprenant deux desdits appareils (30,60), dont
- un premier appareil (30), dans lequel le dispositif de ventilation (40) au moins partiellement contenu dans le premier appareil est constitué du mélangeur oxygène-air à effet venturi connecté à l'ouverture d'entrée (21) de l'interface patient (20) ; et
- un deuxième appareil (60), dans lequel le dispositif de ventilation (70) au moins partiellement contenu dans le deuxième appareil est constitué du clapet d'expiration connecté à l'ouverture d'écoulement (22) de l'interface patient (20).

18. Système (10) selon la revendication précédente, dans lequel le premier appareil (30) comprend des moyens de support amovibles (500) adaptés pour soutenir le deuxième appareil (60).
